# EUROPEAN PATENT APPLICATION

(11) **EP 1 374 904 A1**
(43) Date of publication of application: **02.01.2004**
(21) Application number: 02077529.2
(22) Date of filing: 20.06.2002
(51) Int. Cl.: A61K 47/34, A61P 19/00, A61K 31/70, A61K 7/04, A61K 31/00

(54) **Preparation containing polymethyl-siloxane for nails, cartilage, bone joint, muscle and tendon disorders**

(71) Applicant: Baas, Fhilipus Albert, 7261 JH Ruurlo (NL)
(72) Inventor: Taal, Leendert, 7255 NL Hengelo (GLD) (NL)
(74) Representative: 't Jong, Bastiaan Jacobus

(57) **Abstract**

The present invention relates to a topical preparation that contains polydimethylsiloxane for the external treatment of nail, cartilage, bone, joint, muscle and tendon disorders. The preparation can also contain biotin, zinc, vanadium, lysine, methionine, laurel extract, carotene, casein, collagen, glucose, a glucosamine and/or arnica extract. In particular the invention relates to a combination therapy where a glucosamine is administered orally.

## Description

The present invention relates to a preparation for the treatment of nail, cartilage, bone, joint, muscle and tendon disorders in particular, the invention relates to a preparation for the treatment of lower legs, feet and hooves of horses.

The treatment of nail, cartilage, bone, joint, muscle and tendon disorders is generally carried out by combating the disorder internally of externally at the site of the disorder. In WO 00/15202, for example, it is described that nail disorders can be treated by topical application of an agent that contains an active ingredient in combination with a carrier based on a C1-C4 alkyl ester of lactic acid, malic acid tartaric acid or citric acid. Antibiotics and disinfectants can, for example, be incorporated as active ingredients.

US 4996043 describes a composition for the treatment of keratinous material, in particular horses hooves, which contains an aqueous dispersion containing 20-90% water, oils with a high content of mono-unsaturated fatty acids and water soluble of swellable polysaccharides. Furthermore, a maximum of 10% silicone oil can be added.

The disadvantage of the preparations according to the state of art is that they do not provide adequate transfer of the active component to the surface to be treated. As a result the active component does not penetrate adequately.

Furthermore, in particular in the case of the treatment of horses, it is important that the preparation remains on the location to be treated for some considerable time and is not substantially removed by, for example, walking in pasture.

It has now been found that nail, cartilage, bone, joint, muscle and tendon disorder can be effectively prevented and/or treated by use of a preparation that contains an active ingredient and, as carries, at least 50% (m/m), based on the weight of the preparation, of polydimethylsiloxane. Polydimethylsiloxane is also termed Dimethicone. Dimethicone known to have a number of synonyms; it is known under the names of: Dimethicon(e), Dimethyl Silicone fluid, Dimethylpolysiloxane, Dimethylsiloxane, Dimeticonum, Huile de silicone, Methylpolysiloxane, permetthylpolysiloxane, Siliconeolie, Siliconum liquidum, Poly (dimethylsiloxane), Silicone Rubber; Latex; Dimethylpoly- siloxane; Simethicone; Dimethyl silicone, Dermafilm; Dimethicream; Silbar, is all a compound having the following structural formula;

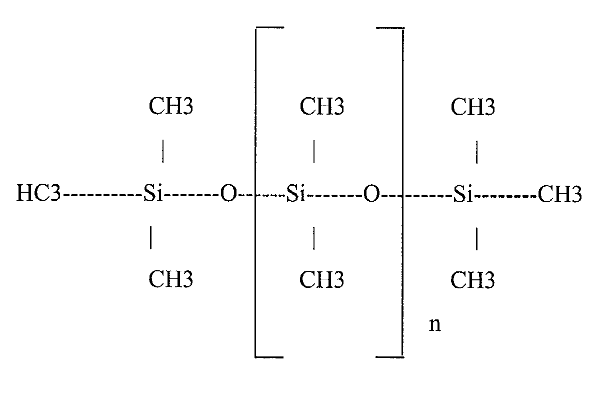

The official name for the substance is Dimethicone.

### Use for Human and veterinary cases must have, by law, a pharmaceutical quality.

Dimethicon for pharmaceutical use has to be compendial (pharmacoporial) grade. Therefore, Dimethicone has a monograph in the European Pharmacopoeia. Furthermore, Dimeticone, pharmaceutical grade is described in the following pharmacopoeia: Belgium, United Kingdom, The Netherlands, France, Greece, Portugal, Switzerland and the British Veterinary Pharmacopoeia.
These monographs are equal to the monograph in the European Pharmacopoeia.

Dimethicone is also described in the Chinese pharmacopoeia and The US national formulary. Demands on viscosity and limits on impurities differ in these monographs for the European monograph.

An important point of distinction, apart from the purity, is the viscosity of the Dimeticon solution. The European Pharmacopoeia describes Dimethicone with a kinematic viscosity of 20-1300 mm2/s. This viscosity is permitted for use by animals and human.

The U.S.N.F. describes a range of 20-30.000 centistokes. Values for kinematic viscosity outside this range, are by definition, not of pharmacopial standard.

Dimethicon is known as an agent for the protection of skin, for example in the case of decubitus, incontinence an dermatoses. It has now been found that these quality's polydimethylsiloxane is an excellent carrier in the case of the treatment of the above mentioned disorders. In particular this agent increases the permeability of the tissue to which it is applied, as a result of which the action of active components that are present in the agent increased.

Preferably, a polydimethylsiloxane having a viscosity of 20-1300 mm2/s preferably 0-10.000 mm2/s, for USNF. Preferably 20-30.000 cSt pharmaceutical quality is used. The preparation preferably contains at lest 70% (m/m) polydimethylsiloxane. Furthermore, the preparation is essentially anhydrous, that is to say it contains less than 20% (m/m), preferably less than 10% (m/m), and most preferentially less than 5% (m/m) water. It is also possible to use an activated polydimethylsiloxane, for example activated with silicon dioxide (simethicone). The percentage of pharmaceutical quality silicon dioxyde can then be approximately 2 to approximately 20% (m/m) based on the total preparation.

The active ingredients incorporated in the preparation of the invention are preferably one or more of the following; biotin, zinc, vanadium, lysine and methionine, preferably a combination of all these substances.

Biotin (hexahydro-2-oxo-1H-thienol {3,4-d} imidazole-4-pentanoic acid) is a growth factor, which is present in small amounts in every cell. The quantity of biotin in the preparation according to the invention is 10 to 100 gram per 400 gram polydimethylsiloxane. Biotin is a basic raw material for the production of keratin (the hoof in horses and nails in man).

Furthermore, stress is reduced, as a result of which reconditioning proceeds more efficiently. Stress has the effect of destroying the growth process. Lysine and methionine are essential amino acids. The preparation according to the invention contains 1 to 20 gram lysine and 1 to 20 gram methionine per 400 gram polydimethylsiloxane. Together with biotin, lysine has an effect on the formation of keratin. Together with methionine forms keratin form the epithelium. Zinc is present in the preparation in an amount of 1 to 10 gram zinc per 400 gram polydimethylsiloxane. Zinc acts as a catalyst which in combination with biotin, lysine and methionine forms a procedures keratin in the epithelium under the influence of oxygen-rich blood.

In addition to the above mentioned active ingredients, one or more of the following can also be added to the preparation: laurel extract, carotene, caseinate, collagen, glucose and arnica extract.

Laurel extract is obtained from the laurel berry, *Laurus nobilis L.* The laurel extract can be in the form of a laurel powder, but can also be a laurel oil. The laurel extract is obtained in the manner known in the art. The preparation according to the invention contains 1 to 30 gram of laurel extract per 400 gram polydimethylsiloxane. Laurel extract promotes the growth maturing from keratin. It also prevents premature aging of tissue, for example in horses in the further outgrowth region of the hoof, it guarantees an improvement in the elasticity of the horn wall.

Carotene, in particular β-carotene, is also referred to as provitamin A. It occurs in, for example, carrots and pal oil. In horses, for example, carotene indirectly and directly produces improved keratin production in the growth region of the hoof, at the transition from the leathery skin to the hoof horn. The preparation according to the invention contains 1 to 20 gram carotene per 400 gram polydimethylsiloxane.

Caseinate of casein is a mixture of phosphorus proteins and has a high content of essential amino acids. It can be obtained from milk in the conventional manner. Preferably (calcium) caseinate from cow's mil is used. The preparation contains 1 to 20 gram caseinate per 400 gram polydimethylsiloxane.

Collagen is a polypeptide which comprises one third of the total protein in vertebrate mammals. It is one of the most important constituents of the skin, tissue and the organic component in bones and teeth. The preparation contains 5 to 30 grams collagen per 400 gram polydimethylsiloxane.

The topical preparation preferably also contains a glucosamine, derivatives thereof and/or salts, which gives particularly favourable results. Preferably an acetylglucosamine is used and more preferentially N-acetyl-D-glucosamine or a pharmaceutically acceptable salt thereof, in particular N-acetyl-D-glucosamine HCI and Glutamine L.

An alternative component that can be incorporated in the topical preparation is arnica extract. This is obtained form *Arnica montana L.* Arnica oil is preferably used in the preparation according to the invention. The quantity of arnica extract is 1 to 10 gram per 400 gram polydimethylsiloxane. It reduces pain in the case of injuries and improves the recovery process.

According to another aspect of the invention three separate topical preparations are provided. The first preparation comprises polydimethylsiloxane and one or more, preferably all, of the following: biotin, lysine, methionine, zinc, caseinate, carotene, laurel powder and glucosamine. This first preparation is in particular suitable for the treatment of nails, hooves, cartilage and joints. The second preparation comprises polydimethylsiloxane and on or more, preferably all, of the following: glucose, collagen, carotene, caseinate, laurel powder and glucosamine. This second preparation is in particular suitable for the treatment of tendons and muscles. The third preparation contains polydimethylsiloxane and arnica extract. This third preparation is in particular suitable for pain control.

The nail, cartilage, bone, joint, muscle and tendon disorders which can be treated using the preparation according to the invention can exist both in humans and in animals. Examples of disorders in humans are rheumatic disorders such as Cushing's syndrome, osteoporosis, rheumatoid arthritis and arthrosis, cartilage degeneration, necrosis, joint disorders, joint mobilisation and impaired growth. The topical agent is applied to the intended location at least once a week. In addition it has been found that the preparation can advantageously be used in the case of tendon and ligament disorders.

It has been found that the preparation according to the invention is particularly suitable for the treatment of disorders in horses, in particular disorder of the hooves, feet and lower legs, most preferentially of hooves.

Examples of disorders which can be treated using the preparation according to the invention are:
- Loose wall, an interruption in the union between the supporting rim of the horn wall and the horn sole in the white line of in some other location;
- Hollow wall, detachment between various laminae of the wall;
- Horny pillar, a cylindrical renewal of the horn in the longitudinal direction on the inside of the horny wall;
- Horn cracks, to be differentiated according to location, extent and depth. They mostly occur on the horn wall. The horn cracks in the wall run in the longitudinal direction, that is to say in the direction of the horn bars. There are said to be bearing surface cracks, coronet cracks and continuous cracks, from the coronet to the bearing surface;
- Navicular disease, inflammation of the navicular bone, the collective name for frog, mucus bursa and coffin bone flexor tendon. Chronic inflammation of this area is termed chronic navicular disease (Podotrochleitis chronica) ;
- Crumbly hoof;
- Hard hooves;
- Red frog;
- Cancer of the frog; and
- Hoof cancer

In addition to the topical application of the abovementioned preparation based on polydimethylsiloxane, it has been found that combination with an oral preparation that contains a glucosamine, derivatives thereof and/or salts gives particularly favourable results. Preferably, an acetylglucosamine, more preferentially N-acetyl-D-glucosamine or a pharmaceutically acceptable salt thereof, in particular N-acetyl-D-glucosamine HCI, is used. The quantity of glucosamine per day is 100 to 2,500 mg, preferably 1,000 to 2,000 mg.

The combination of the topical preparation with a glucosamine is in particular suitable when improvement in the cartilage and protection and build up of the joint is desired. In addition this combination can be used to improve the function of reconditioning of tendons and muscles.

The oral preparation that contains a glucosamine can be, for example, a capsule, tablet, powder or drink solution. It contains a carries suitable for the type of dosage form. In particular, the oral preparation that contains a glucosamine is a tablet with, as carrier, one or more of the following: gelatine, magnesium stearate, sugar and lactose.

The topical preparation according to the preparation can have, for example, the following compositions:

| **Preparation 1** | |
|---|---|
| (in particular suitable for the treatment of nails, cartilage, bones and hooves) polydimethylsiloxane (dimethicone, viscosity 0-10.000 mm2/s) | 400 g |
| biotin | 60 g |
| lysine | 7.5 g |
| methionine | 7.5 g |
| zinc | 2.3 g |
| vanadium | 2.1 g |
| caseinate | 4 g |
| carotene | 2 g |
| laurel powder | 28 g |
| N-acetyl-D-glucosamine HCI + Glutamine L | 1.5 g |

| **Preparation 2** | |
|---|---|
| (in particular suitable for treatment of joints, muscles and tendons) polydimethylsiloxane (dimethicone, viscosity 0-10.000 mm2/s) | 400 g |
| glucose syrup | 40 g |
| collagen | 20 g |
| carotene | 10 g |
| caseinate | 10 g |
| laurel powder | 10 g |
| N-acetyl-D-glucosamine HCI + Glutamine L | 1.5 g |

| **Preparation 3** | |
|---|---|
| Polydimethylsiloxane (dimethicone, viscosity preferably 20-1300 mm2/s more and 0-10.000 cSt USNF 20-30.000 cSt pharmaceutical quality. | 400 g |
| arnica oil | 6.5 g |

When treating with the topical preparation the latter is applied to the site of the disorder. Fresh preparation is applied regularly, for example once a week. The treatment continues as long as is necessary to heal the disorder. In general this will be a period of 1 to 6 months.

The oral preparation containing glucosamine is preferably administered in combination with the topical preparation. The oral preparation is administered daily, for example in a dosage of 1 gram per day. Optionally this daily dose can be split into several doses.

### Treatment

Smear hoof ointment (Preparation 1) into coronet once per week. Oral administration of powder stopped.

In a corresponding manner, the use of the hoof ointment (Preparation 1), on its own or in combination with the oral preparation, was found to be suitable for the treatment of loose wall, tilting of the coffin bone, horny pillar, seizure of the hoof mechanism and ball arthrosis.

Treatment: Smearing with tendon ointment (Preparation 2) and 1 gram per day N-acetyl-D-glucosamine HCI and Glutamine L, administered orally.

## Claims

1. Topical preparation that contains an active ingredient and, as carrier, at least 50% (m/m), based on the weight of the preparation, of polydimethylsiloxane for the external treatment of nail, cartilage, bone, joint, muscle and tendon disorders.

2. Preparation according to Claim 1, wherein the polydimethylsiloxane has a viscosity of 20-1300 mm2/s (0-1300mm2/s preferably and 0-10.000 mm2/s for USNF 20-30.000 cSt)

3. Preparation according to Claim 1 or 2, wherein the active ingredient is chosen from biotin, zinc, lysine, methionine and mixtures thereof.

4. Preparation according to Claim 3, that contains biotin, zinc, lysine and methionine.

5. Preparation according to one of the preceding claims, that further contains a glucosamine, derivatives thereof and/or salts thereof, preferably N-acetyl-D-glucosamine of a pharmaceutically acceptable salt thereof, more preferentially N-acetyl-D-Glucosamine HCI and Glutamine L.

6. Preparation according to one of the preceding claims that further contains one or more of the following: laurel extract, carotene, casein, collagen, glucose and arnica extract.

7. Preparation according to Claim 1, that contains biotin, lysine, methionine zinc, casein, carotene, arnica extract, laurel extract and N-acetyl-D-glucosamine HCI and Glutamine L.

8. Preparation according to Claim 1, that contains glucose, collagen, carotene, casein, laurel extract and N-acetyl-D-glucosamine HCI and Glutamine L.

9. Preparation according to Claim 1, that contains arnica extract.

10. Preparation according to one of the preceding claims for the treatment of horses.

11. Preparation according to Claim 10 for the treatment of disorders of the hooves, feet and lower legs of horses.

12. Preparation according to Claim 11, for the treatment of horses'hooves.

13. Preparation according to one of Claims 1 to 8 for the treatment of humans.

14. Use of an oral preparation that contains a glucosamine, derivatives thereof and/or salts thereof in combination with the preparation according to one of the preceding claims for the treatment of nail, cartilage, bone, joint, muscle and tendon disorders.

15. Use according to Claim 14, wherein the oral preparation contains N-acetyl-D-glucosamine of a pharmaceutically acceptable salt thereof, preferably N-acetyl-D-glucosamine HCI and Glutamine L.

16. Kit comprising an oral preparation that contains a glucosamine and topical preparation that contains polydimethylsiloxane as a combination for simultaneous, separate or successive administration.

17. Kit according to Claim 16, for the treatment of nail, cartilage, bone, joint, muscle and tendon disorders.

18. Use of polydimethylsiloxane having an pharmaceutical quality and preferably 0-10.000 mm2/s a viscosity of 20-1300mm2/s cSt and USNF 20-30.000 cSt pharmaceutical quality as carrier in a topical preparation, wherein the preparation contains at least 50% (m/m), based on the weight of the preparation, of polydimethylsiloxane.

19. Use according to Claim 18, wherein the topical preparation is used for the external treatment of nails, cartilage, bones, joint, muscles and tendons.

20. Use according to Claim 18 or 19, wherein the topical preparation contains at least 50% (m/m) polydimethylsiloxane.
